# EUROPEAN PATENT SPECIFICATION

(11) **EP 3 849 519 B1**
(45) Date of publication and mention of the grant of the patent: **21.09.2022**
(21) Application number: 19773254.8
(22) Date of filing: 06.09.2019
(51) Int. Cl.: A61K 9/08, A61K 39/395, A61K 47/14

(54) **A COMPOSITION COMPRISING A PROTEIN AND A POLYALKOXY FATTY ACYL SURFACTANT**
ZUSAMMENSETZUNG ENTHALTEND EIN PROTEIN UND EINEM POLYALKOXY-FETTSÄURE-TENSID
COMPOSITION COMPRENANT UNE PROTÉINE ET UN TENSIOACTIF ACYLE GRAS POLYALKOXYLENE

(30) Priority: 10.09.2018 US 201862729140 P; 21.05.2019 US 201962850688 P
(43) Date of publication of application: 21.07.2021
(73) Proprietor: DOW GLOBAL TECHNOLOGIES LLC, Midland, MI 48674 (US)
(72) Inventor: KATZ, Joshua S., Wilmington, Delaware 19805 (US); JORDAN, Susan L., Wilmington, Delaware 19805 (US)
(74) Representative: DuPont EMEA
(86) International application number: PCT/US2019/049904
(87) International publication number: WO 2020/055679

(56) References cited:
- WO-A1-2017/044367
- DE-A1-102005 011 608

## Description

### FIELD

The present invention relates to compositions comprising a protein and a polyoxyalkyl fatty acyl surfactant.

### INTRODUCTION

Biologics, pharmaceuticals derived from proteins or other biologically-derived macromolecules, have rapidly emerged as an important class of pharmaceuticals. Due to the relatively fragile nature of protein materials, development of biologic actives that are both therapeutically beneficial and sufficiently stable to withstand processing, distribution, and administration remains a significant challenge. Protein biologic drug producers and formulators employ a variety of process techniques and formulation strategies to improve the shelf-stability of formulations, but the current toolbox of excipients is fairly small. Dry powders of proteins are often more shelf-stable so many first generation product offerings are dried, either by lyophilization or, more recently, spray drying. However, dry products can have challenges relating to reconstitution, leading to insoluble species that may cause immunogenicity. Further, reconstitution is a process that typically requires a medical professional to perform, leading to increased costs and inconvenience for the patient. Many protein drugs are delivered in the clinic through IV infusion, particularly for oncology drugs. As protein drugs are developed for other diseases, such as rheumatoid arthritis or other autoimmune disorders, patients prefer self-administration at home. With the move from the clinic to the home, liquid formulations, where feasible, are the preferred delivery form.

To improve liquid formulation stability, formulators employ various excipients designed to stabilize the biologic, including buffers, salts, sugars, and surfactants. These excipients are used to optimize the environment around the protein to maximize its colloidal stability, reduce interactions with neighboring proteins, and block interactions with surfaces. Surfactants in particular stabilize proteins by blocking the proteins' access to hydrophobic surfaces (e.g. vial walls or air interfaces) on which they can adsorb, denature, and ultimately aggregate. For some biologics, surfactants have also been proposed to stabilize certain protein sub-regions or block protein-protein interactions that could also lead to denaturation, though this is not believed to be the dominant mechanism of action. The vast majority of products formulated with surfactants contain either a polysorbate (Polysorbate 80 or Polysorbate 20) or Poloxamer 188, as they have well-established safety profiles.

While polysorbates and Poloxamer188 have been utilized because of their safety in injectable formulations, they still have significant drawbacks, especially due to surfactant degradation. A series of surfactants that are built on common, inert building blocks for use in protein formulations has recently been developed as disclosed in WO 2017/044366. These novel surfactants comprise a fully-saturated alkyl chain, commonly found in natural lipids, an amino acid residue that could fine-tune the functionality of the surfactant, and a polyether non-ionic hydrophilic head group. These building blocks were combined through amide bonds to resist hydrolysis of the hydrophobe (compared to an ester bond), which has been implicated in polysorbate degradation. The use of these surfactants to stabilize proteins in aqueous solution is disclosed in WO 2017/044367. The concentration of the surfactant relative to the protein reported in the formulations of WO 2017/044367 is 1:200 or less and preferably 1:100 or less.

### SUMMARY OF THE INVENTION

It has surprisingly been found that surfactants disclosed in WO 2017/044366 can be used to prevent aggregation of proteins in aqueous solutions at much lower concentrations relative to the protein concentration than those reported in WO 2017/044367.

Accordingly, the present invention relates to a composition comprising a protein and a polyalkoxy fatty acyl surfactant compound of general formula I 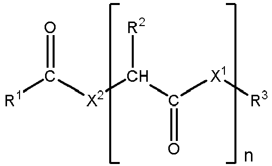 wherein R¹-C(=O) is a fatty acyl group, R² is H or a substituted or unsubstituted hydrocarbyl group, X¹ is O or NH, X² is O or NH, n is 0 or an integer of 1-5, R³ is a polymeric group comprising polymerized units of general formula II and III 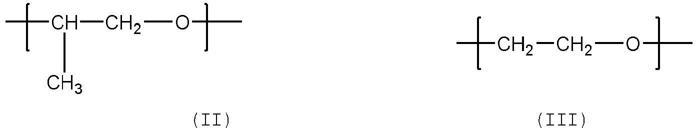 wherein the weight ratio of said protein to said polyalkoxy fatty acyl surfactant is in the range of 200:1 to 1000:1.

Surfactants of formula (I) have been found to exhibit a critical micelle concentration (the concentration above which a surfactant spontaneously assembles into micelles) that is lower than that of conventional surfactants such as polysorbates or Poloxamer 188. A lower critical micelle concentration is indicative of a stronger drive to assemble which may translate into a faster stabilization of an interface. Without wishing to be limited to a particular theory, a lower critical micelle concentration may explain the finding that surfactants of formual (I) approach surface equilibrium 1 to 2 orders of magnitude faster than the conventional surfactants and outcompete a protein at an interface, thus reducing the likelihood of protein aggregation.

### DETAILED DESCRIPTION OF THE INVENTION

### Definitions

A polyalkoxy compound is a compound that contains one or more group having the structure -(-A-O)ₘ-, where m is three or more, and A is an unsubstituted alkyl group. The group A may be linear, branched, cyclic, or a combination thereof. The various A groups among the various -(-A-O)- groups may be the same as each other or different.

A fatty compound is a compound that contains one or more fatty group. A fatty group is a group that contains 8 or more carbon atoms, each of which is bonded to one or more of the other carbon atoms in the group. A polyalkoxy fatty compound is a compound that is both a polyalkoxy compound and a fatty compound.

Number-average molecular weight is defined as the total weight of a sample divided by the number of molecules in the sample.

A hydrocarbyl group is a group that contains hydrogen and carbon atoms. An unsubstituted hydrocarbyl group contains only hydrogen and carbon atoms. A substituted hydrocarbyl group contains one or more substituent group that contains one or more atom other than hydrogen and carbon.

A protein is a polymer in which the polymerized units are polymerized units of amino acids. The amino acids are bonded together by peptide bonds. A protein contains 20 or more polymerized units of one or more amino acid residues. The term protein includes linear polypeptide chains as well as more complex structures that contain polypeptide chains.

A protein is considered to be in solution in a liquid medium (or, synonymously, dissolved in the liquid medium) if the molecules of the protein are distributed throughout the continuous liquid medium in the form of dissolved individual molecules. The protein is considered to be dissolved in water if the continuous liquid medium contains water in the amount of 60% or more by weight based on the weight of the continuous liquid medium.

A chemical group is an ionic group if there is a pH value between 4.5 and 8.5 such that, when the chemical group is in contact with water at that pH value, 50 mole% or more of those chemical groups present are in ionic form.

A buffer is either (i) a compound that has the ability to accept a proton to form the conjugate acid of that compound, and the conjugate acid of that compound has pKa of less than 10, or (ii) a compound that has the ability to release a proton, and the compound has pKa of greater than 4.

### Embodiments

In the polyalkoxy fatty compound of formula I, R¹ is preferably a substituted or unsubstituted aliphatic group. Among substituted aliphatic groups, preferred substituent is hydroxyl. More preferably R¹ is an unsubstituted aliphatic group; more preferably, R¹ is an unsubstituted alkyl group. Preferably, R¹ is a linear alkyl group with 9-22 carbon atoms, preferably 10-18 carbon atoms, more preferably 10-16 carbon atoms.
Preferably, X¹ is NH. Preferably, X² is NH
Preferably, n is 0 or 1, 2, 3, 4 or 5. More preferably, n is 0 or 1.
Preferably, R² has 20 or fewer atoms; more preferably 15 or fewer. Preferably, if R² is not hydrogen, then R² contains one or more carbon atom. Preferably, R² is either hydrogen or an unsubstituted hydrocarbon group; more preferably, R² is either hydrogen, an unsubstituted alkyl group, or an alkyl group whose only substituent is an unsubstituted aromatic hydrocarbon group. Among unsubstituted alkyl groups, preferred is methyl. Among alkyl groups whose only substituent is an unsubstituted aromatic hydrocarbon group, preferred is -CH₂-(C₆H₅), where -(C₆H₅) is a benzene ring. Preferably, R² represents a side chain of a naturally occurring amino acid.
Preferably, R³ has a number-average molecular weight of 600-5000 Daltons, more preferably 800-3000 Daltons. Preferably, the group R³ is either a statistical copolymer of (II) and (III) or a block copolymer of (II) and (III); more preferably the group R³ is a statistical copolymer of (II) and (III). Preferably, -R³ has the structure -R⁴-CH₃, where R⁴ is a polymeric group comprising polymerized units of structure (II) and structure (III). Preferably, R⁴ has no other polymerized units in addition to structure (II) and (III).

It is useful to characterize the mole ratio (herein the "PO/EO ratio") of units of structure (II) to units of structure (III). Preferably, the PO/EO ratio is 0.01:1 to 2:1; more preferably 0.05:1 to 1:1, in particular 0.1:1 to 0.5:1.

In a particularly preferred embodiment of the compound of formula (I), R¹ is CH₃-(CH₂)₁₁-CH₂-, n is 1, X¹ and X² are both NH, R² is -CH₂(C₆H₅), and R³ is a copolymer of PO and EO units capped with CH₃ with an approximate number-average molecular weight of 1000 and ratio of PO to EO of 3:19. This surfactant has been found to stabilize an interface 1 to 2 orders of magnitude faster than polysorbates or Poloxamer 188 which are the standard surfactants used to stabilize aqueous protein solutions. The faster dynamics have been found to lead to improved stabilization of model protein drugs against agitation induced aggregation.

In another preferred embodiment of the compound of formula (I), R¹ is CH₃-(CH₂)₁₁-CH₂-, n is 0, X² is NH, and R³ is a copolymer of PO and EO units capped with CH₃ with an approximate number-average molecular weight of 1000 and ratio of PO to EO of 3:19.

Preferably, the compound of formula (I) has no ionic groups.

The compound of formula (I) may be made by any method. A preferred method is to react a compound having structure NH₂-R³ with a compound selected from compounds of structure V 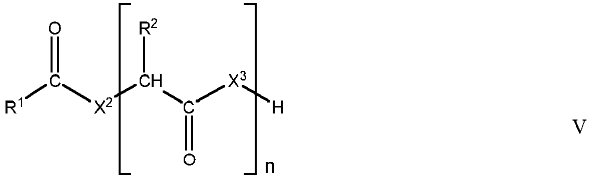 and compounds of structure VI 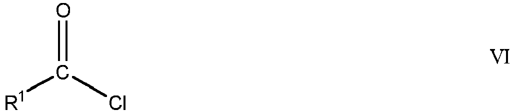 where X³ is O or NH If n is 1-5, each X³ is independently O or NH. The terminal X³ is preferably O. Preferences for R¹, X², R², R³, and n are the same as those described above. Preferably, X³ is O.

Another preferred method of making some embodiments of the compound of formula (I) is as follows. In a first step, an acyl chloride is reacted with an amino acid to form a carboxyl-functional fatty amide as follows:

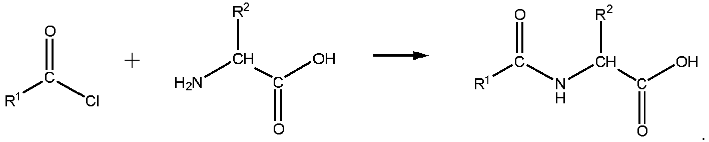

Then, in a second step, the carboxyl-functional fatty amide is reacted with an amine-terminated polyalkoxy compound, as follows: 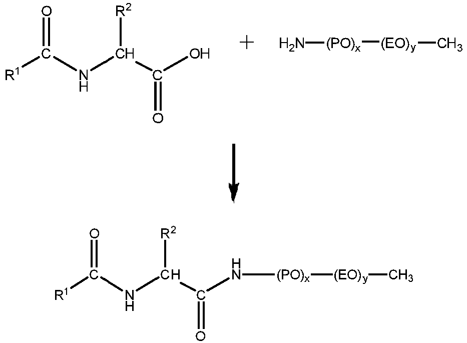 where PO is structure (II) and EO is structure (III). It should be noted that when R³ is shown as (PO)ₓ and (EO)_{y} in the structures above, it should not be taken as an indication that the PO and EO units invariably are present as blocks; indeed, they may also be randomly distributed throughout R³.

Preferred proteins to be included in the present composition are selected from monoclonal antibodies, growth factors, insulins, immunoglobulins, polyclonal antibodies, antibody-drug conjugates, hormones, enzymes, polypeptides, fusions of peptides, glycosylated proteins, antigens, antigen subunits, or combinations thereof. Preferred proteins have therapeutic efficacy to treat a disease or medical condition or to function as vaccines. Examples of therapeutic proteins are immunoglobulin-g, adalimumab, interferon alfa, bevacizumab, human growth hormone, rituximab, human serum albumin, insulin, erythropoietin alpha, pembrolizumab, etanercept, filgrastim, nivolumab, trastuzumab, durvalumab, interleukin-2, infliximab, chorionic gonadotropin, avelumab, denosumab, ranibizumab, aflibercept, tremelimumab, factor viii, interferon beta, ipilimumab, atezolizumab, abatacept, tocilizumab, ustekinumab, pegfilgrastim, secukinumab, streptokinase, cetuximab, omalizumab, ramucirumab, urokinase, certolizumab pegol, dupilumab, genolimzumab, aldesleukin, molgramostim, peginterferon alfa-2b, tislelizumab, follitropin alfa, gevokizumab, golimumab, spartalizumab, canakinumab, foralumab, varlilumab, nimotuzumab, erythropoietin beta, evolocumab, pegargiminase, bermekimab, carotuximab, daratumumab, eculizumab, ontuxizumab, adalimumab, camrelizumab, enoblituzumab, interleukin-12, lirilumab, panitumumab, gatipotuzumab, relatlimab, andecaliximab, belimumab, cabiralizumab, isactuzumab govitecan, monalizumab, pancreatin, pertuzumab, toripalimab, inebilizumab, ofatumumab, pepinemab, sintilimab, alirocumab, milatuzumab, nidanilimab, sotatercept, vedolizumab, veltuzumab, bevacizumab beta, isatuximab, orlotamab, tisotumab vedotin, benralizumab, cosibelimab, emactuzumab, ganitumab, narsoplimab, pidilizumab, sarilumab, trastuzumab emtansine, anetumab ravtansine, bertilimumab, blinatumomab, guselkumab, ixekizumab, mepolizumab, obinutuzumab, ublituximab, alemtuzumab, emibetuzumab, ficlatuzumab, ifabotuzumab, mirikizumab, natalizumab, racotumomab, siltuximab, timigutuzumab, trastuzumab deruxtecan, bimekizumab, brodalumab, cetrelimab, farletuzumab, opinercept, rilonacept, tomuzotuximab, urelumab, ascrinvacumab, brolucizumab, clazakizumab, cusatuzumab, dalotuzumab, ianalumab, itolizumab, and margetuximab. Also contemplated are proteins that can be used as medical diagnostics or have a beneficial effect on a food composition, or be incorporated in a cleaning composition or a coatings formulation.

Preferably, the weight ratio of protein to compound of formula (I) is in the range of 250:1 to 900:1, more preferably in the range of 300:1 to 800:1, in particular in the range of 400:1 to 700:1. Alternatively, the weight ratio of protein to compound of formula (I) is in the range of 201:1 to 250:1, preferably in the range of 205:1to 245:1, more preferably in the range of 210:1 to 240:1.

In a particularly preferred embodiment, the present invention relates to a composition as described above wherein the monoclonal antibody is cetuximab and wherein the ratio of cetuximab to polyalkoxy fatty acyl surfactant of formula (I) is from 300:1 to 750:1. In this embodiment, the polyalkoxy fatty acyl surfactant is preferably a compound of formula (I) wherein R¹ is CH₃-(CH₂)₁₁-CH₂-, n is 1, X¹ and X² are both NH, R² is -CH₂(C₆H₅), and R³ is a copolymer of PO and EO units capped with CH₃ with an approximate number-average molecular weight of 1000 and ratio of PO to EO of 3:19.

A preferred method of making a composition that contains both a protein and a compound of formula (I) is to mix together water, one or more proteins, one or more compounds of formula (I), and optional additional ingredients to provide a composition in which the protein is dissolved in water.

In the present composition, preferably the protein molecules are not aggregated into large particles, even if the aggregated particles are dispersed in the liquid medium. Preferably, if any aggregated particles that contain protein molecules are present, the volume-average hydrodynamic radius of such particles is 10 nm or smaller; more preferably 6 nm or smaller.

Preferably, the amount of protein in the present composition is in the range of 0.01 mg/ml to 400 mg/ml, preferably 0.1 mg/ml to 300 mg/ml, more preferably 1 mg/ml to 250 mg/ml.

The present composition optionally contains one or more additional ingredients. Additional ingredients are compounds other than water, proteins, and compounds of formula (I).

Preferred additional ingredients are sugars, sugar alcohols, salts, buffers, amino acids, salts of amino acids, and mixtures thereof. When such additional ingredients are present, preferably the total amount of all additional ingredients is 300 mg/mL or less.

For inclusion in the present composition, preferred sugars are sucrose, glucose, mannose, trehalose, maltose, dextrose, dextran and mixtures thereof. Preferred sugar alcohols for inclusion in the present composition are sorbitol, mannitol or xylitol.

For inclusion in the present composition, preferred salts have cations chosen from hydrogen, sodium, potassium, magnesium, calcium, ammonium, and mixtures thereof. Preferred salts have anions chosen from fluoride, chloride, bromide, iodide, phosphate, carbonate, acetate, citrate, sulfate, and mixtures thereof. Preferred buffers have cations chosen from hydrogen, sodium, potassium, magnesium, calcium, ammonium, and mixtures thereof. Preferred buffers have anions chosen from fluoride, chloride, bromide, iodide, phosphate, carbonate, acetate, citrate, sulfate, and mixtures thereof.

For inclusion in the present composition, preferred amino acids and salts thereof are selected from lysine, glycine, arginine, histidine, and mixtures thereof.

The invention is further described in the follwing examples which are not in any way intended to limit the scope of the invention as claimed.

### EXAMPLES

### Materials

Unless otherwise noted, all materials were received from Sigma-Aldrich or Fisher and used without further purification. Jeffamine M-1000 was provided by Huntsman. Polysorbate 80 (PS80) and Polysorbate 20 (PS20) were the "tested according to Ph.Eur." grade, Sigma-Aldrich product numbers 59924 and 44112, respectively. They were stored under a nitrogen headspace to minimize oxidation. Poloxamer 188 (PO188) was purchased as a 10% sterile-filtered aqueous solution, Sigma product number P5556. Industrial grade bovine IgG was purchased from MP Biomedicals (Santa Ana, CA).

FM1000 is a surfactant compound of formula (I), wherein R¹ is CH₃-(CH₂)₁₁-CH₂-, n is 1, X¹ and X² are both NH, R² is -CH₂(C₆H₅), and R³ is a copolymer of PO and EO units capped with CH₃ with an approximate molecular weight of 1000 and ratio of PO to EO of 3:19. FM1000 was prepared as reported in WO 2017/044366. Briefly, myristoyl chloride was amidated with phenylalanine in water in the presence of sodium hydroxide and triethylamine. The resulting suspension was acidified to pH 2 with concentrated HCl and filtered. The filtered powder was then recrystallized from hexanes. The myristoyl phenylalanine was amidated by melt condensation with Jeffamine M-1000. The crude FM1000 product was dissolved in methanol and stirred over Dow Amberlite IRN 77 and IRN78 ion exchange resins to remove starting materials. The final product was dried under vacuum.

OM1000 is a surfactant compound of formula (I), wherein R¹ is CH₃-(CH₂)₁₁-CH₂-, n is 0, X¹ is NH and R³ is a copolymer of PO and EO units capped with CH₃ with an approximate molecular weight of 1000 and ratio of PO to EO of 3:19. OM1000 was prepared as reported in WO 2017/044366.

### Methods

Samples were prepared with a concentration of 20 mg/mL IgG in 0.9% aqueous sodium chloride. Surfactant concentrations are listed in the Examples. Dynamic Light Scattering (DLS) measurements were performed as follows. Light scattering measurements were performed on a Wyatt DynaPro^{™} high throughput DLS instrument. 30 µL of each sample were placed in the wells of Corning 96-well plates. The plates were centrifuged for 3 minutes at 1000 rpm before analysis. The samples were allowed to equilibrate at 25°C for 5 minutes before analysis. DLS measurements were obtained continuously, cycling from well to well with 5 x 3 sec acquisitions per well per cycle.

### Example 1

600 µL Formulations were prepared in 1 mL glass vials. 100 µL were removed for t=0 analysis and then the sample was shaken on its side at 200 rpm overnight at 35 degrees C. Phase clarity was measured for each vial and a 100 µL aliquot was measured for size via DLS. Data are shown in the following tables:

**Phase Clarity:**

| Surfactant Concentratio n (mg/mL) | PS80 | PS20 | PO188 | FM1000 |
|---|---|---|---|---|
| T=0 | 13 | 13.33 | 13.66 | 14 |
| 0.2 | 17 | 16 | 45 | 14 |
| 0.1 | 19 | 17 | 50 | 14 |
| 0.075 | 19 | 21 | 54 | 16 |
| 0.05 | 31 | 20 | 56 | 17 |
| 0.03 | 43 | 31 | 49 | 26 |
| 0.02 | 39 | 36 | 58 | 45 |
| 0.01 | 47 | 46 | 57 | 51 |
| 0 | 53 | 46 | 47 | 46 |
| 0.2 | 17 | 15 | 48 | 12 |
| 0.1 | 20 | 17 | 51 | 15 |
| 0.075 | 19 | 19 | 53 | 16 |
| 0.05 | 28 | 21 | 54 | 19 |
| 0.03 | 39 | 30 | 51 | 26 |
| 0.02 | 41 | 38 | 54 | 40 |
| 0.01 | 45 | 39 | 58 | 54 |
| 0 | 47 | 47 | 47 | 45 |
| 0.2 | 18 | 15 | 43 | 14 |
| 0.1 | 23 | 17 | 47 | 15 |
| 0.075 | 21 | 18 | 47 | 15 |
| 0.05 | 33 | 25 | 51 | 22 |
| 0.03 | 44 | 33 | 49 | 28 |
| 0.02 | 39 | 37 | 57 | 40 |
| 0.01 | 45 | 40 | 58 | 56 |
| 0 | 45 | 43 | 46 | 49 |

**Light scattering data, average size (nm)**

| Surfactant Concentratio n (mg/mL) | PS80 | PS20 | PO188 | FM1000 |
|---|---|---|---|---|
| 0.2 | 10.1 | 8.6 | 89.1 | 7.5 |
| 0.1 | 11.2 | 10 | 94.2 | 7.9 |
| 0.075 | 11.7 | 10.9 | 84 | 8.3 |
| 0.05 | 28.2 | 14.4 | 79.3 | 9.5 |
| 0.03 | 53.1 | 26.7 | 78.3 | 17.6 |
| 0.02 | 48.4 | 36 | 200 | 200 |
| 0.01 | 46.2 | 30.2 | 200 | 200 |
| 0.001 | 200 | 200 | 200 | 200 |
| 0.2 | 10 | 8.7 | 96 | 7.3 |
| 0.1 | 12.5 | 10.2 | 87 | 8 |
| 0.075 | 11.9 | 11.4 | 88.8 | 8.4 |
| 0.05 | 26.8 | 13.8 | 72.3 | 9.8 |
| 0.03 | 54.4 | 28.5 | 93.7 | 17.9 |
| 0.02 | 200 | 42.5 | 200 | 200 |
| 0.01 | 52 | 27.3 | 68.4 | 200 |
| 0.001 | 200 | 81 | 200 | 200 |
| 0.2 | 10.3 | 8.5 | 84.9 | 7.5 |
| 0.1 | 12.6 | 10.1 | 83.7 | 7.8 |
| 0.075 | 13.6 | 10.9 | 81.2 | 8.2 |
| 0.05 | 37.2 | 18.2 | 93.9 | 11.5 |
| 0.03 | 62 | 31.8 | 79.6 | 18.7 |
| 0.02 | 52.4 | 31 | 200 | 200 |
| 0.01 | 52.1 | 30.6 | 200 | 109.4 |
| 0.001 | 200 | 200 | 200 | 200 |
| T=0 | 7.1 | 7.2 | 7.1 | 7.2 |
| T=0 | 6.9 | 7.1 | 7 | 6.9 |
| T=0 | 7.2 | 6.9 | 7.2 | 7 |

From the light scattering data, the concentration required to keep the average radius below a certain size can be calculated:

| | Concentration to reach radius of 14 | | |
|---|---|---|---|
| | PS80 | PS20 | FM1000 |
| mg/mL | 0.111 | 0.069 | 0.038 |
| +/- | 0.011 | 0.004 | 0.002 |
| | | | |

| | Concentration to reach radius of 12 | | |
|---|---|---|---|
| | PS80 | PS20 | FM1000 |
| mg/mL | 0.129 | 0.087 | 0.050 |
| +/- | 0.013 | 0.005 | 0.003 |

### Example 2: Kinetics of Aggregation

The experiment was set up as in Example 1 but at a set concentration of surfactant: 0.05 mg/mL (ratio of 400:1). At each time point, samples were taken off the shaker and analyzed for hydrodynamic radius via light scattering. Value of 100 was inputted for un-readable data.

**Hydrodynamic Radii.**

| Shake Time (hr) | PS80 | PS20 | PO188 | FM1000 | Control |
|---|---|---|---|---|---|
| 0 | 7.7 | 7.9 | 7.8 | 8 | 100 |
| 0 | 7.7 | 8 | 7.9 | 7.9 | 100 |
| 0 | 8 | 7.9 | 8 | 8.1 | 100 |
| 1 | 7.7 | 7.7 | 9.4 | 7.8 | 7.9 |
| 1 | 7.4 | 7.7 | 9 | 7.7 | 8.2 |
| 1 | 7.6 | 7.4 | 8.9 | 7.6 | 100 |
| 2 | 10.3 | 8.2 | 20.8 | 7.6 | 10.3 |
| 2 | 8.7 | 8.2 | 14.1 | 7.9 | 9.1 |
| 2 | 8.6 | 7.9 | 11.7 | 7.9 | 100 |
| 4 | 14.5 | 8.6 | 100 | 8.2 | 12.1 |
| 4 | 11.9 | 9.4 | 100 | 8 | 11.7 |
| 4 | 14.4 | 8.8 | 100 | 8.1 | 100 |
| 6 | 100 | 10 | 100 | 9.2 | 13.9 |
| 6 | 21.3 | 11.3 | 100 | 8.4 | 16.2 |
| 6 | 11.5 | 10 | 100 | 10.4 | 100 |

This data can be fit and used to calculate the time to reach a given radius measured by DLS:

| Time to reach size (hr): | | | | | |
|---|---|---|---|---|---|
| Size (nm) | PS80 | PS20 | PO188 | FM1000 | Control |
| 12 | 2.739808 | 9.47214 | 1.086454 | 17.3755 | 4.077699 |
| 16 | 4.024676 | 15.2839 | 1.663318 | 28.3978 | 6.467085 |
| 20 | 5.021298 | 19.79185 | 2.110769 | 36.94736 | 8.320436 |

### Example 3

The experiment was set up as in Example 1, but a polyalkoxy fatty acyl surfactant of formula (I) containing no amino acid residue (OM1000, n=0, X1=NH₂) was used as the excipient and compared to FM1000. The concentration of IgG was 20 mg/mL; the surfactant concentration was 0.05 mg/mL (ratio of protein to surfactant was 400:1). Polysorbate 20 was also studied as a control. 500 µL samples were shaken as in the other examples for 18 hrs. The data are as follows:

| | Clarity | Abs(350nm) | Radius |
|---|---|---|---|
| FM1000 | 13.7 | 0.194 | 8.29 |
| OM1000 | 13.3 | 0.191 | 8.36 |
| PS20 | 19.7 | 0.209 | ^{∗} |

| | | | |
|---|---|---|---|
| (^{∗}: no radius could be measured for this experiment) | | | |

### Example 4

The experiment was set up as in Example 3, but the solution contained 25 mM citrate (pH 6) rather than 0.9% sodium chloride. The concentration of IgG was 20 mg/mL; the surfactant concentration was 0.05 mg/mL (ratio of protein to surfactant was 400:1). Clarity was not measured for these samples. The data are as follows:

| Surfactant | Absorbance (350nm) | Radius (nm) |
|---|---|---|
| FM1000 | 0.100 | 8.07 |
| OM1000 | 0.116 | 9.81 |
| PS20 | 0.137 | 17.73 |
| PS80 | 0.112 | 10.21 |

These data show that FM1000 and OM1000 are more effective than polysorbates at stabilizing IgG at high protein to surfactant ratio in a low ionic strength buffer.

### Example 5

### Surfactant effect on stability of cetuximab

Cetuximab is commercially available under the trade name Erbitux^{®} and was acquired from a pharmacy. It is formulated as a 2 mg/mL solution in 10 mM phosphate buffer and 145 mM sodium chloride, pH 7.2.

Microflow imaging was performed on a Biotechne Microflow Imaging unit to quantify subvisible particles. Particle counts were automated by the software. Size exclusion chromatography was run on an Agilent 1260 Bioinert liquid chromatography system with UV detection. The column was an Agilent AdvanceBio 300A SEC column and running buffer was phosphate buffer. UV absorbance was measured on a Molecular Devices M3 Plate reader. Dynamic Light Scattering (DSC) was measured on a Wyatt DynaPro II.

Cetuximab was reformulated to contain varying amounts of FM1000 or Polysorbate 80. 1.5 mg/mL cetuximab samples were diluted directly from Erbitux (2 mg/mL) to the final formulation. The 7.5 mg/mL samples were concentrated via centrifugal filtration to 10 mg/mL and then diluted to 7.5 mg/mL. Surfactant stock solutions were prepared in 10 mM phosphate, 145 mM NaCl, pH 7.2. Each shaken sample was 3.0 mL in a 5 mL serum vial from Wheaton. Pre-shaken samples were aliquots removed to bring the shaken sample volume to 3 mL. Shaken samples were shaken overnight at 150 strokes/min on a reciprocal shaker.

| Experiment # | [CTX] (mg/mL ) | [Surf] (mg/mL ) | Surfactant | Shake? | Radius (nm) | Particle Count | GPC AUC |
|---|---|---|---|---|---|---|---|
| 1 | 1.5 | 0.075 | FM1000 | Pre-Shake | 6.4 | | 612.7 |
| 2 | 1.5 | 0.075 | PS80 | Pre-Shake | 5.5 | | 623.8 |
| 3 | 1.5 | 0.05 | FM1000 | Pre-Shake | 6.1 | | 640.6 |
| 4 | 1.5 | 0.05 | PS80 | Pre-Shake | 5.5 | | 639.6 |
| 5 | 1.5 | 0.02 | FM1000 | Pre-Shake | 5.9 | | 636.7 |
| 6 | 1.5 | 0.02 | PS80 | Pre-Shake | 5.6 | | 621.2 |
| 7 | 1.5 | 0.01 | FM1000 | Pre-Shake | 5.8 | | 610.7 |
| 8 | 1.5 | 0.01 | PS80 | Pre-Shake | 5.5 | | 616.8 |
| 9 | 1.5 | 0.005 | FM1000 | Pre-Shake | 5.6 | | 611.5 |
| 10 | 1.5 | 0.005 | PS80 | Pre-Shake | 5.5 | | 613.6 |
| 11 | 1.5 | 0.075 | FM1000 | Post-Shake | 6.4 | 7335 | 634.8 |
| 12 | 1.5 | 0.075 | PS80 | Post-Shake | 6.2 | 3069 | 626.4 |
| 13 | 1.5 | 0.05 | FM1000 | Post-Shake | 6.2 | 6137 | 615.0 |
| 14 | 1.5 | 0.05 | PS80 | Post-Shake | 6.2 | 5077 | 623.3 |
| 15 | 1.5 | 0.02 | FM1000 | Post-Shake | 6.1 | 7134 | 622.5 |
| 16 | 1.5 | 0.02 | PS80 | Post-Shake | 6.2 | 8937 | 625.6 |
| 17 | 1.5 | 0.01 | FM1000 | Post-Shake | 5.7 | 3803 | 605.2 |
| 18 | 1.5 | 0.01 | PS80 | Post-Shake | 8.9 | 29418 | 598.8 |
| 19 | 1.5 | 0.005 | FM1000 | Post-Shake | 7.0 | 6310 | 573.3 |
| 20 | 1.5 | 0.005 | PS80 | Post-Shake | 14.6 | 264211 | 511.6 |
| 21 | 1.5 | 0.0025 | FM1000 | Pre-Shake | 5.7 | | 596.5 |
| 22 | 1.5 | 0.001 | PS80 | Pre-Shake | 5.6 | | 587.7 |
| 23 | 1.5 | 0.0025 | FM1000 | Post-Shake | 10.9 | 630466 | 457.8 |
| 24 | 1.5 | 0.001 | PS80 | Post-Shake | 0.0 | 832005 | 434.0 |
| 25 | 7.5 | 0.01 | FM1000 | Pre-Shake | 6.3 | | 3025.1 |
| 26 | 7.5 | 0.01 | PS80 | Pre-Shake | 6.3 | | 3025.2 |
| 27 | 7.5 | 0.01 | FM1000 | Post-Shake | 6.3 | 777 | 3068.8 |
| 28 | 7.5 | 0.01 | PS80 | Post-Shake | 6.8 | 3874 | 3013.1 |
| 29 | 7.5 | 0 | none | Pre-Shake | | 861 | |
| 30 | 7.5 | 0 | none | Post-Shake | | 628344 | |
| 31 | 7.5 | 0.02 | FM1000 | Post-Shake | | 8310 | |
| 32 | 7.5 | 0.02 | PS80 | Post-Shake | | 8521 | |
| 33 | 7.5 | 0 | none | Pre-Shake | | 1442 | |

It appears from the table above that at a level of 1.5 mg/ml cetuximab and 0.075 mg/ml or 0.05 mg/ml surfactant, the particle count was lower when polysorbate 80 was included as the surfactant. At these levels of surfactant, the particle size (radius nm) was not significantly different between the two surfactants and between pre- and post-shaken samples. At 0.02 mg/ml surfactant, neither the particle count nor the particle size were significantly different between the two surfactants.

At a level of 1.5 mg/ml cetuximab and 0.01 mg/ml of either polysorbate 80 or FM1000 (ratio of protein to surfactant of 150:1), the particle count was lower when FM1000 was included as the surfactant as was the particle size.

At a level of 1.5 mg/ml cetuximab and 0.005 mg/ml of either polysorbate 80 or FM1000 (ratio of protein to surfactant of 300:1), the particle count was much lower when FM1000 is included as the surfactant, and the particle size was also significantly smaller.

At a level of 7.5 mg/ml cetuximab and 0.01 mg/ml of either polysorbate 80 and FM1000 (ratio of protein to surfactant of 750:1), the particle count was lower when FM1000 was included as the surfactant.

These results show that FM1000 can be used as a surfactant to reduce the formation of cetuximab particles to near-baseline levels after significant agitation stress even at a very low level of the surfactant.

## Claims

1. A composition comprising a protein and a polyalkoxy fatty acyl surfactant of general formula I 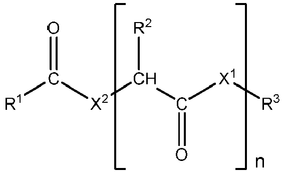 wherein R¹-C(=O) is a fatty acyl group, R² is H or a substituted or unsubstituted hydrocarbyl group, X¹ is O or NH, X² is O or NH, n is 0 or an integer of 1-5, R³ is a polymeric group comprising polymerized units of general formula II and III 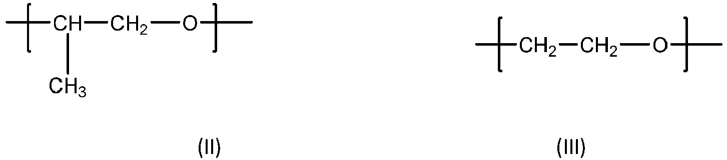 wherein the weight ratio of said protein to said polyalkoxy fatty acyl surfactant is in the range of 200:1 to 1000:1.

2. The composition of claim 1, wherein the weight ratio of said protein to said polyalkoxy fatty acyl surfactant is in the range of 250:1 to 900:1.

3. The composition of claim 1 or 2, wherein the weight ratio of said protein to said polyalkoxy fatty acyl surfactant is in the range of 300:1 to 800:1.

4. The composition of any one of claims 1-3, wherein the weight ratio of said protein to said polyalkoxy fatty acyl surfactant is in the range of 400:1 to 700:1.

5. The composition of claim 1, wherein the weight ratio of protein to said polyalkoxy fatty acyl surfactant is in the range of 201:1 to 250:1.

6. The composition of claim 5, wherein the weight ratio of protein to said polyalkoxy fatty acyl surfactant is in the range of 205:1 to 245:1.

7. The composition of claim 5 or 6, wherein the weight ratio of protein to said polyalkoxy fatty acyl surfactant is in the range of 210:1 to 240:1.

8. The composition of any one of claims 1-7, wherein X² is NH

9. The composition of any one of claims 1-8, wherein n is 1, 2, 3, 4 or 5, preferably 1.

10. The composition of any one of claims 1-9, wherein X¹ is NH

11. The composition of any one of claims 1-10, wherein, in formula I, R¹ is CH₃-(CH₂)₁₁-CH₂-, n is 1, X¹ and X² are both NH, R² is -CH₂(C₆H₅), and R³ is a copolymer of PO and EO units capped with CH₃ with an approximate molecular weight of 1000 and ratio of PO to EO of 3:19.

12. The composition of any one of claims 1-10, wherein, in formula I, R¹ is CH₃-(CH₂)₁₁-CH₂-, n is 0, X² is NH, and R³ is a copolymer of PO and EO units capped with CH₃ with an approximate molecular weight of 1000 and ratio of PO to EO of 3:19.

13. The composition of any one of claims 1-12, wherein the amount of protein is in the range of 0.01 mg/ml to 400 mg/ml, preferably 0.1 mg/ml to 300 mg/ml, more preferably 1 mg/ml to 250 mg/ml.

14. The composition of any one of claims 1-13, wherein the protein is cetuximab.

15. The composition of claim 14, wherein the ratio of cetuximab to polyalkoxy fatty acyl surfactant is from 300:1 to 750:1.

## Patentansprüche

1. Zusammensetzung, umfassend ein Protein und ein Polyalkoxyfettacyl-Tensid der allgemeinen Formel I 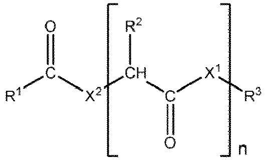 wobei R¹-C(=O) für eine Fettacylgruppe steht, R² für H oder eine substituierte oder unsubstituierte Hydrocarbylgruppe steht, X¹ für O oder NH steht, X² für O oder NH steht, n für 0 oder eine ganze Zahl von 1-5 steht, R³ für eine polymere Gruppe, die polymerisierte Einheiten der allgemeinen Formel II und III 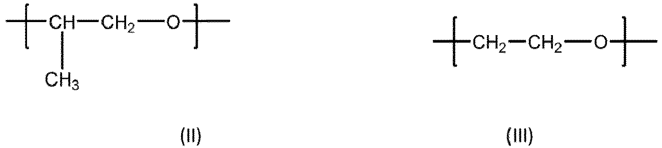 umfasst, steht,
wobei das Gewichtsverhältnis von Protein zu Polyalkoxyfettacyl-Tensid im Bereich von 200:1 bis 1000:1 liegt.

2. Zusammensetzung nach Anspruch 1, wobei das Gewichtsverhältnis von Protein zu Polyalkoxyfettacyl-Tensid im Bereich von 250:1 bis 900:1 liegt.

3. Zusammensetzung nach Anspruch 1 oder 2, wobei das Gewichtsverhältnis von Protein zu Polyalkoxyfettacyl-Tensid im Bereich von 300:1 bis 800:1 liegt.

4. Zusammensetzung nach einem der Ansprüche 1-3, wobei das Gewichtsverhältnis von Protein zu Polyalkoxyfettacyl-Tensid im Bereich von 400:1 bis 700:1 liegt.

5. Zusammensetzung nach Anspruch 1, wobei das Gewichtsverhältnis von Protein zu Polyalkoxyfettacyl-Tensid im Bereich von 201:1 bis 250:1 liegt.

6. Zusammensetzung nach Anspruch 5, wobei das Gewichtsverhältnis von Protein zu Polyalkoxyfettacyl-Tensid im Bereich von 205:1 bis 245:1 liegt.

7. Zusammensetzung nach Anspruch 5 oder 6, wobei das Gewichtsverhältnis von Protein zu Polyalkoxyfettacyl-Tensid im Bereich von 210:1 bis 240:1 liegt.

8. Zusammensetzung nach einem der Ansprüche 1-7, wobei X² für NH steht.

9. Zusammensetzung nach einem der Ansprüche 1-8, wobei n für 1, 2, 3, 4 oder 5, vorzugsweise 1, steht.

10. Zusammensetzung nach einem der Ansprüche 1-9, wobei X¹ für NH steht.

11. Zusammensetzung nach einem der Ansprüche 1-10, wobei in Formel I R¹ für CH₃-(CH₂)₁₁-CH₂- steht, n für 1 steht, X¹ und X² beide für NH stehen, R² für -CH₂(C₆H₅) steht und R³ für ein mit CH₃ verkapptes Copolymer von PO- und EO-Einheiten mit einem ungefähren Molekulargewicht von 1000 und einem Verhältnis von PO zu EO von 3:19 steht.

12. Zusammensetzung nach einem der Ansprüche 1-10, wobei in Formel I R¹ für CH₃-(CH₂)₁₁-CH₂- steht, n für 0 steht, X² für NH steht und R³ für ein mit CH₃ verkapptes Copolymer von PO- und EO-Einheiten mit einem ungefähren Molekulargewicht von 1000 und einem Verhältnis von PO zu EO von 3:19 steht.

13. Zusammensetzung nach einem der Ansprüche 1-12, wobei die Menge an Protein im Bereich von 0,01 mg/ml bis 400 mg/ml, vorzugsweise 0,1 mg/ml bis 300 mg/ml, weiter bevorzugt 1 mg/ml bis 250 mg/ml, beträgt.

14. Zusammensetzung nach einem der Ansprüche 1-13, wobei es sich bei dem Protein um Cetuximab handelt.

15. Zusammensetzung nach Anspruch 14, wobei das Verhältnis von Cetuximab zu Polyalkoxyfettacyl-Tensid 300:1 bis 750:1 beträgt.

## Revendications

1. Composition comprenant une protéine et tensioactif de type acyle gras polyalcoxy de formule générale I 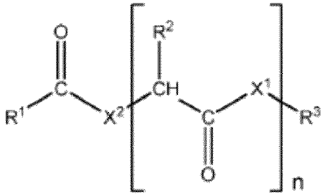 R¹-C(=O) étant un groupe acyle gras, R² étant H ou un groupe hydrocarbyle substitué ou non substitué, X¹ étant O ou NH, X² étant O ou NH, n étant 0 ou un entier de 1 à 5, R³ étant un groupe polymérique comprenant des motifs polymérisés de formule générale II et III 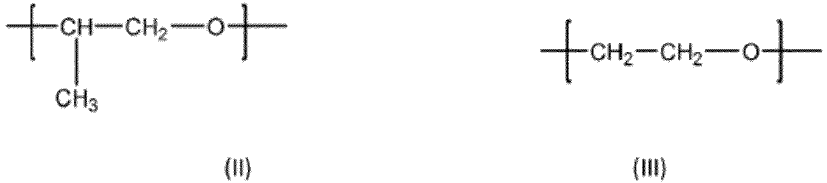 le rapport en poids de ladite protéine sur ledit tensioactif de type acyle gras polyalcoxy étant dans la plage de 200 : 1 à 1 000 : 1.

2. Composition selon la revendication 1, le rapport en poids de ladite protéine sur ledit tensioactif de type acyle gras polyalcoxy étant dans la plage de 250 : 1 à 900 : 1.

3. Composition selon la revendication 1 ou 2, le rapport en poids de ladite protéine sur ledit tensioactif de type acyle gras polyalcoxy étant dans la plage de 300 : 1 à 800 : 1.

4. Composition selon l'une quelconque des revendications 1 à 3, le rapport en poids de ladite protéine sur ledit tensioactif de type acyle gras polyalcoxy étant dans la plage de 400 : 1 à 700 : 1.

5. Composition selon la revendication 1, le rapport en poids de la protéine sur ledit tensioactif de type acyle gras polyalcoxy étant dans la plage de 201 : 1 à 250 : 1.

6. Composition selon la revendication 5, le rapport en poids de la protéine sur ledit tensioactif de type acyle gras polyalcoxy étant dans la plage de 205 : 1 à 245 : 1.

7. Composition selon la revendication 5 ou 6, le rapport en poids de la protéine sur ledit tensioactif de type acyle gras polyalcoxy étant dans la plage de 210 : 1 à 240 : 1.

8. Composition selon l'une quelconque des revendications 1 à 7, X² étant NH.

9. Composition selon l'une quelconque des revendications 1 à 8, n étant 1, 2, 3, 4 ou 5, préférablement 1.

10. Composition selon l'une quelconque des revendications 1 à 9, X¹ étant NH.

11. Composition selon l'une quelconque des revendications 1 à 10, dans laquelle, dans la formule I, R¹ est CH₃-(CH₂)₁₁-CH₂-, n est 1, X¹ et X² sont tous deux NH, R² est -CH₂(C₆H₅), et R³ est un copolymère de motifs PO et EO coiffés par CH₃ dotés d'un poids moléculaire approximatif de 1 000 et d'un rapport de PO sur EO de 3 : 19.

12. Composition selon l'une quelconque des revendications 1 à 10, dans laquelle, dans la formule I, R¹ est CH₃-(CH₂)₁₁-CH₂-, n est 0, X² est NH, et R³ est un copolymère de motifs PO et EO coiffés par CH₃ dotés d'un poids moléculaire approximatif de 1 000 et d'un rapport de PO sur EO de 3 : 19.

13. Composition selon l'une quelconque des revendications 1 à 12, la quantité de protéines étant dans la plage de 0,01 mg/ml à 400 mg/ml, préférablement de 0,1 mg/ml à 300 mg/ml, plus préférablement de 1 mg/ml à 250 mg/ml.

14. Composition selon l'une quelconque des revendications 1 à 13, la protéine étant cétuximab.

15. Composition selon la revendication 14, le rapport de cétuximab sur le tensioactif de type acyle gras polyalcoxy étant de 300 : 1 à 750 : 1.
